# EUROPÄISCHE PATENTANMELDUNG

(11) **EP 2 314 343 A1**
(43) Veröffentlichungstag der Anmeldung: **27.04.2011**
(21) Anmeldenummer: 10188368.4
(22) Anmeldetag: 21.10.2010
(51) Int. Cl.: A61M 39/20

(54) **Verschlusskappen für Fluidverbindungssysteme**

(30) Priorität: 23.10.2009 DE 202009013409 U
(71) Anmelder: Hopf, Hans-Jürgen, 90513 Zirndorf (DE)
(72) Erfinder: Hopf, Hans-Jürgen, 90513 Zirndorf (DE); Kassai, Norbert, 90522 Oberasbach (DE); Hopf, Alexander, 90491 Nürnberg (DE); Hopf, Michael, 90513 Zirndorf (DE)
(74) Vertreter: Fleuchaus, Michael A.

(57) **Zusammenfassung**

Die vorliegende Erfindung betrifft eine Verschlusskappe für ein verriegelbares Verbindungssystem für fluiddurchströmte Bauteile für die Medizin und Medizintechnik mit einem männlichen, konusförmigen Steckelement (22,42,62), welches zentrisch innen liegend in einem äußeren Fixierabschnitt (10,20,30,40,50,60) angeordnet ist, wobei der äußere Fixierabschnitt das männliche, konusförmige Steckelement in seiner Längserstreckung vollständig umfasst.

## Beschreibung

Die vorliegende Erfindung betrifft Verschlusskappen für Anschlusssysteme an Fluidverbindungen, wie sie insbesondere in der Medizin und Medizintechnik eingesetzt werden.

Anschlusssysteme für Fluidverbindungen sind im Stand der Technik bekannt und werden insbesondere in der Medizin und Medizintechnik eingesetzt. Hierbei werden die Anschlusssysteme insbesondere im Infusionsbereich, dem Bereich der künstlichen Ernährung, bei Spritzen und Injektionsbesteck, bei der Transfusion und insbesondere für die Zu- bzw. Überleitung von verschiedenen Durchflussmedien und als sogenanntes "Injektions-Equipment" für die medizinische und pharmazeutische Ausrüstung verwendet, welche unter anderem aus mehreren Komponenten bestehen. Neben den verschiedenen anschließbaren, durchfließbaren Komponenten gibt es auch mit den Anschlusssystemen kompatible Verschlusssysteme in Form von sogenannten Verschlusskappen.

In der Medizin ist die absolute Sicherheit in der Bedienung von Fluidverbindungen oberstes Gebot. Daher hat sich in der Medizintechnik das Luer bzw. Luer-Lock Prinzip gegenüber früher gebräuchlichen Verbindungssystemen durchgesetzt.

Luer-Lock ist ein genormtes Verbindungssystem für Kanülen, Spritzen und Infusions-Schläuche im medizinischen Bereich.

Die Dichtung wird hierbei durch eine kegelförmige Konstruktion der Verbindungsteile, des sogenannten Luer-Konus, erreicht. Dabei wird der Innenkegel der einen

Verbindungsseite auch als "weiblich" bezeichnet, der Außenkegel der Gegenseite als "männlich". Zur Sicherung bzw. Verriegelung der Verbindung gegen versehentliches Lösen ist der Konus durch ein Gewinde erweitert. Verschlusskappen sind gemeinhin derart gestaltet, dass ein dem männlichen Anschluss entsprechender Außenkegel in den weiblichen Anschluss gedrückt wird und mit Hilfe der Überwurfmutter fixiert wird.

Die nötige Dichtheit wird dadurch erreicht, dass bei gleicher Steigung des Innenkonus und des Außenkonus die in ihrem Radius korrespondierenden Abschnitte der beiden Verbindungsteile aneinander gedrückt werden. Dies bedeutet, dass führ eine entsprechende Verbindung neben der Steigung auch der Radius der Koni und damit auch ihre Länge respektive Tiefe von Bedeutung ist. So kann durch längere Verbindungselemente eine höhere Toleranz an den jeweiligen Verbindungspartner erreicht werden, da ein größeres Radienspektrum innerhalb der Koni vorhanden ist. Dies hat unter Umständen zur Folge, dass die räumliche Ausdehnung der Koni über die jeweiligen Fixierabschnitte hinaus geht. Im Fall von Verschlusskappen mit männlichen Verbindungselementen steht dann der Konus aus dem Fixierabschnitt hervor.

Jedoch ergibt sich durch den aus der Verschlusskappe hervorstehenden Konus eine erhöhte Kontaminationsgefahr. Der männliche Dichtkonus der Verschlusskappe kommt funktionsbedingt im Betrieb mit dem Fluid des Fluidsystems in Kontakt. Durch den aus der Überwurfmutter hervorstehenden Konus kann dieser leicht bei der Montage mit nicht sterilem Material in Kontakt kommen, wodurch Keime in das System eingebracht werden können.

Aufgabe der vorliegenden Erfindung ist es, die im Stand der Technik bekannten Nachteile, insbesondere die Kontaminationsgefahr des Systems bei gleichbleibender Dichtheit wenigstens teilweise zu reduzieren.

Gelöst wird die vorstehende Aufgabe durch eine erfindungsgemäße Verschlusskappe für ein Verbindungssystem gemäß Anspruch 1, sowie der Verwendung des Verbindungssystems in der Medizin und Medizintechnik. Bevorzugte Ausgestaltungsformen des Verbindungssystems, sowie der entsprechenden männlichen und weiblichen Verbindungspartner sind Gegenstand der entsprechenden Unteranspruche.

Die erfindungsgemäße Verschlusskappe für den Einsatz in der Medizin und Medizintechnik weist ein männliches, konusförmiges Steckelement auf, das zentrisch innen liegend in einem umgebenden, äußeren Fixierabschnitt angeordnet ist. Die erfindungsgemäße Verschlusskappe ist **dadurch gekennzeichnet, dass** der umgebene, äußere Fixierabschnitt das männliche, konusförmige Steckelement wenigstens in seiner Längserstreckung vollständig umfasst. Das männliche, konusförmige Steckelement steht demnach nicht aus dem umgebenden, äußeren Fixierabschnitt vor.

Als Fixierabschnitt im Sinne der vorliegenden Erfindung werden Vorrichtungen zur Sicherung bzw. Verriegelung der Verbindung verstanden.

Gemäß einer weiteren, besonders bevorzugten Ausführungsform der erfindungsgemäßen Verschlusskappe ist der äußere Fixierabschnitt als Überwurfmutter ausgestaltet und weist ein Innengewinde auf.

Gemäß einer weiteren, besonders bevorzugten Ausführungsform der erfindungsgemäßen Verschlusskappe weist der als Überwurfmutter ausgestaltete äußere Fixierabschnitt einen gewindefreien, hülsenartigen vorderen Bereich auf. Hierdurch wird die Montage der Verschlusskappe an dem jeweiligen Verbindungspartner erleichtert, da die Hülse als Führung bei der Montage dient. Dies ist insbesondere von Bedeutung, da die Führung durch das männliche, konusförmige Steckelement aufgrund der Verkürzung desselben bei der erfindungsgemäßen Verschlusskappe nicht gegeben ist.

Gemäß einer weiteren, besonders bevorzugten Ausführungsform der erfindungsgemäßen Verschlusskappe weist das männliche, konusförmige Steckelement eine Steigung von 6 % auf.

Gemäß einer weiteren, besonders bevorzugten Ausführungsform der erfindungsgemäßen Verschlusskappe weist diese eine wenigstes teilweise elastische Öse auf, welche im Außenbereich der Verschlusskappe angeordnet ist. Durch diese Öse kann die Verschlusskappe mit dem jeweiligen Verbindungspartner fixiert werden. Gemäß einer weiteren, besonders bevorzugten Ausführungsform ist die Öse über einen wenigstens teilweise elastischen, länglichen Abschnitt mit der Verschlusskappe verbunden.

Gemäß einer weiteren, besonders bevorzugten Ausführungsform weist die Öse eine weitere Öse auf, mittels derer die Öse reversibel mit der Verschlusskappe verbunden ist.

Durch die Öse in den oben beschriebenen Ausführungsformen kann die Verschlusskappe an den jeweiligen Verbindungspartnern der Verschlusskappe angebracht werden, wodurch eine Verliersicherung der Verschlusskappe bewirkt wird.

Gemäß einer weiteren, bevorzugten Ausführungsform der erfindungsgemäßen Verschlusskappe sind wenigstens Teile der Verschlusskappe aus einem Material hergestellt, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Kombinationen hiervon und dergleichen umfasst.

Die vorliegende Erfindung umfasst ferner auch die Verwendung der erfindungsgemäßen Verschlusskappe in der Medizin und Medizintechnik, insbesondere für den Verschluss verschiedener Leitungskomponenten für die Zu- und Überleitung von verschiedenen Durchflussmedien, insbesondere für die Schwerkraftinfusion, für Pumpüberleitungssysteme, Sondennahrungssysteme, Injektionen sowie Kombinationen hiervon und dergleichen.

Die Erfindung wird nachfolgend anhand eines bevorzugten Ausführungsbeispiels erläutert, wobei darauf hingewiesen wird, dass durch dieses Beispiel Abwandlungen beziehungsweise Ergänzungen wie sie sich für den Fachmann unmittelbar ergeben mit umfasst sind. Darüber hinaus stellt dieses bevorzugte Ausführungsbeispiel keine Beschränkung der Erfindung in der Art dar, dass Abwandlungen und Ergänzungen im Umfang der vorliegenden Erfindung liegen.

Dabei zeigen:
Fig. 1 eine Seitenansicht einer erfindungsgemäße Verschlusskappe;
Fig. 2 einen Querschnitt durch eine erfindungsgemäße Verschlusskappe;
Fig. 3 eine Seitenansicht einer erfindungsgemäße Verschlusskappe mit Aufnahmebereich für eine Öse;
Fig. 4 einen Querschnitt durch eine erfindungsgemäße Verschlusskappe mit Aufnahmebereich für eine Öse;
Fig. 5 eine erfindungsgemäße Verschlusskappe mit einer hülsenförmigen Verlängerung des Fixierabschnitts;
Fig. 6 einen Querschnitt durch eine erfindungsgemäße Verschlusskappe mit einer hülsenförmigen Verlängerung des Fixierabschnitts.

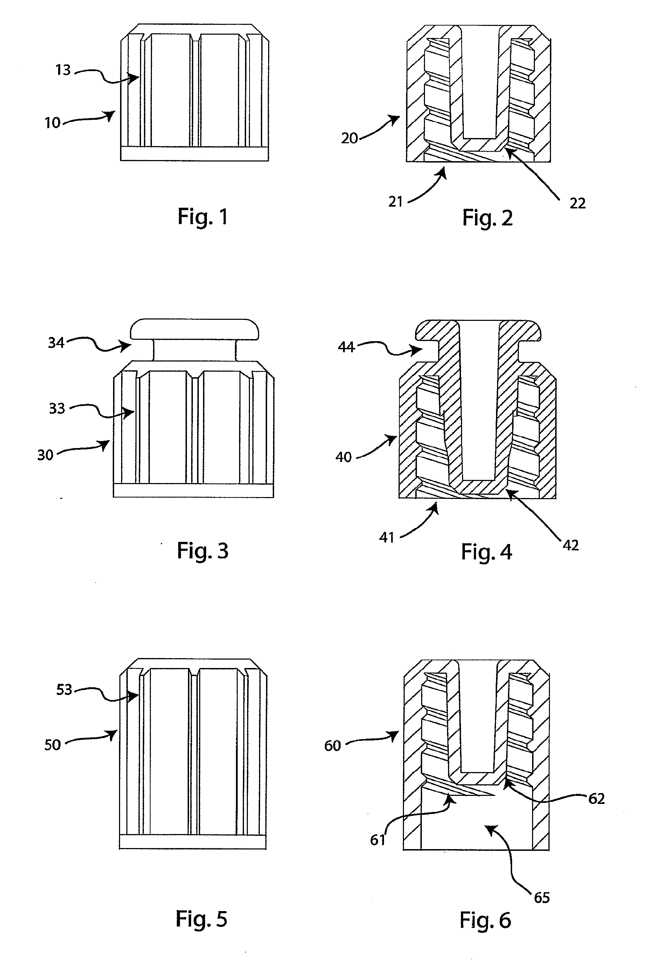

Das Ausführungsbeispiel gemäß Figur 1 und Figur 2 zeigt eine erfindungsgemäße Verschlusskappe bestehend aus einem umgebenen, äußeren Fixierabschnitt 10, 20 der als Überwurfmutter ausgestaltet ist und entsprechend ein Innengewinde 21 und an der Außenseite Strukturelemente 13 zur verbesserten Griffigkeit aufweist. Zentrisch in dem als Überwurfmutter ausgestalteten, äußeren Fixierabschnitt ist das als Konus ausgestaltete männliche, rohrförmige Steckelement 22 angeordnet. Bei der Verwendung der Verschlusskappe greift das männlich, rohrförmige Steckelement in ein weibliches Gegenstück, welches einen entsprechenden Aufnahmeabschnitt ausweist, eines entsprechenden Verbindungssystems ein. Das weibliche Gegenstück weist darüber hinaus einen Fixierabschnitt auf, welcher mit dem als Überwurfmutter ausgestalteten Fixierabschnitt der erfindungsgemäßen Verschlusskappe korrespondiert und somit ein entsprechendes Außengewinde aufweist. Das männlich, konusförmige Steckelement 22 ist erfindungsgemäß so ausgestaltet, dass es von dem umgebenden äußeren Fixierabschnitt vollständig umfasst wird. Es steht als nicht aus dem als Überwurfmutter 10, 20 ausgestalteten Fixierabschnitt hervor. Dadurch wird effektiv verhindert, dass das Steckelement 22 bei der Montage der Verschlusskappe mit unsterilem Material in Berührung kommt und damit kontaminiert und somit Keime in das Fluidsystem eingebracht werden.

In einem weiteren Ausführungsbeispiel weist die erfindungsgemäße Verschlusskappe wie sie in den Figuren 1 und 2 beschrieben ist darüber hinaus eine Öse auf, welche vorzugsweise am äußeren Rand der Verschlusskappe angeordnet ist. Mittels dieser Öse kann die Verschlusskappe an dem jeweiligem Gegenstück, wie zum Beispiel an einem Mehrweghahn angebracht werden, so dass die Verschlusskappe auch wenn sie nicht verschließend montiert ist an dem entsprechenden Element verliersicher angeordnet ist. Hierdurch kann zum einen zusätzlich eine Kontamination der Verschlusskappe, zum Beispiel durch Herabfallen auf den Boden, verhindert werden. Zum anderen ist die einzeln relativ kleine Verschlusskappe an ein größeres Element angebracht und kann somit nicht verloren oder gar von Kleinkindern verschluckt werden. Dieses Ausführungsbeispiel trägt somit gesteigerten Sicherheitsanforderungen Rechnung.

In einem weiteren Ausführungsbeispiel ist eine Öse über einen wenigstens teilweise elastischen, länglichen Abschnitt mit der Verschlusskappe verbunden. Dadurch kann die Öse leichter an dem jeweiligen Verbindungspartner der Verschlusskappe angebracht werden.

Das Ausführungsbeispiel gemäß Figur 3 und Figur 4 zeigt eine erfindungsgemäße Verschlusskappe, bestehend aus einem umgebenen, äußeren Fixierabschnitt 30, 40 der als Überwurfmutter ausgestaltet ist und entsprechend ein Innengewinde 41 und an der Außenseite Strukturelemente 33 zur verbesserten Griffigkeit aufweist. Zentrisch in dem als Überwurfmutter ausgestalteten, äußeren Fixierabschnitt ist das als Konus ausgestaltete männliche, rohrförmige Steckelement 42 angeordnet, welche darüber hinaus einen Aufnahmebereich 34, 44 für eine Öse aufweist. An dieser Aufnahme 34. 44 kann eine Öse befestigt werden, welche eine weitere Öse zur Verbindung mit dem jeweiligen Verbindungspartner der Verschlusskappe aufweist. Die beiden Ösen als Bestandteil beispielsweise eines länglichen, elastischen Verbinders stellen ein flexibles, 8-förmiges Verbindungselement zur Verbindung der Verschlusskappe mit dem jeweiligen Verbindungspartner dar, wodurch die Verschlusskappe auch im geöffneten Zustand an dem jeweiligen Verbindungspartner gehalten wird. Die beiden Ösen können direkt oder über einen wenigstens teilweise elastischen Bereich miteinander verbunden sein.

Das Ausführungsbeispiel gemäß Figur 5 und Figur 6 zeigt eine erfindungsgemäße Verschlusskappe, bestehend aus einem umgebenen, äußeren Fixierabschnitt 50, 60 der als Überwurfmutter ausgestaltet ist und entsprechend ein Innengewinde 61 und an der Außenseite Strukturelemente 53 zur verbesserten Griffigkeit aufweist. Zentrisch in dem als Überwurfmutter ausgestalteten, äußeren Fixierabschnitt ist das als Konus ausgestaltete männliche, rohrförmige Steckelement 62 angeordnet. Der äußere Fixierabschnitt 50, 60 weist eine gewindefreie, hülsenförmige Verlängerung 65 auf. Dieser dient zum einen dem Schutz des männlichen Steckelements 62 und zum anderen als Führungshilfe bei der Montage.

## Patentansprüche

1. Verschlusskappe für ein verriegelbares Verbindungssystem für fluiddurchströmte Bauteile für die Medizin und Medizintechnik mit
einem männlichen, konusförmigen Steckelement (22, 42, 62), welches zentrisch innen liegend in einem äußeren Fixierabschnitt (10, 20, 30, 40, 50, 60) angeordnet ist, **dadurch gekennzeichnet, dass** der
äußere Fixierabschnitt (10, 20, 30, 40, 50, 60) das männliche, konusförmige Steckelement (22, 42, 62) in seiner Längserstreckung vollständig umfasst.

2. Verschlusskappe nach Anspruch 1, **dadurch gekennzeichnet, dass** der äußere Fixierabschnitt (10, 20, 30, 40, 50, 60) eine Überwurfmutter mit einem Innengewinde ist (21, 41, 61).

3. Verschlusskappe nach Anspruch 2, **dadurch gekennzeichnet, dass** der als Überwurfmutter gestaltete äußere Fixierabschnitt (10, 20, 30, 40, 50, 60) vor dem Innengewinde (21, 41, 61) einen gewindefreien, hülsenartigen Bereich aufweist (65).

4. Verschlusskappe nach Anspruch 1 oder 2, **dadurch gekennzeichnet, dass** das männlich, konusförmige Steckelement (22, 42, 62) eine Steigung von 6 % aufweist.

5. Verschlusskappe nach einem der vorstehenden Ansprüche, **dadurch gekennzeichnet, dass**
die Verschlusskappe eine wenigstens teilweise elastische Öse aufweist.

6. Verschlusskappe nach Anspruch 5, **dadurch gekennzeichnet, dass**
das die Öse über einen wenigstens teilweise elastischem länglichen Abschnitt mit der Verschlusskappe verbunden ist.

7. Verschlusskappe nach einem der Ansprüche 5 oder 6, **dadurch gekennzeichnet, dass**
die Öse mittels einer weiteren Öse an der Verschlusskappe befestigt ist.

8. Verschlusskappe nach Anspruch 7, **dadurch gekennzeichnet, dass**
die Verschlusskappe einen Aufnahmebereich (34, 44) zur Aufnahme der Öse aufweist.

9. Verschlusskappe gemäß einem der vorstehenden Ansprüche, **dadurch gekennzeichnet, dass**
das wenigstens Teile der Verschlusskappe aus einem Material hergestellt sind, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, ungesättigtes Polyesterharz, Kombinationen hiervon und dergleichen umfasst.

10. Verwendung einer Vorrichtung gemäß einem der vorstehenden Ansprüche in der Medizin oder Medizintechnik.
